(19) Europäisches Patentamt
European Patent Office
Office européen des brevets

(11) Numéro de publication : **0 509 913 A1**

# (12) DEMANDE DE BREVET EUROPEEN

(21) Numéro de dépôt : **92401052.3**

(51) Int. Cl.$^5$ : **A61K 31/505**

(22) Date de dépôt : **15.04.92**

(30) Priorité : **18.04.91 FR 9104788**

(43) Date de publication de la demande :
**21.10.92 Bulletin 92/43**

(84) Etats contractants désignés :
**AT BE CH DE DK ES FR GB GR IT LI LU MC NL PT SE**

(71) Demandeur : **Aranda, Bernard**
**5 rue Eugène Manuel**
**F-75116 Paris (FR)**

(72) Inventeur : **Aranda, Bernard**
**5 rue Eugène Manuel**
**F-75116 Paris (FR)**

(74) Mandataire : **Warcoin, Jacques et al**
**Cabinet Régimbeau 26, avenue Kléber**
**F-75116 Paris (FR)**

## (54) Utilisation de dérivés et d'analogues du piribedil dans le traitement des vessies hyperactives ou instables.

(57) La présente invention concerne l'utilisation d'un composé de formule générale I

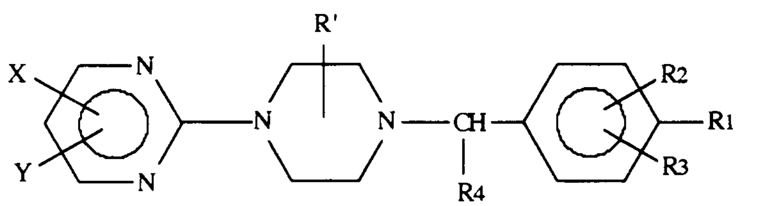

dans laquelle $R_1$, $R_2$, $R_3$, pris séparément ou en combinaison peuvent représenter un atome d'hydrogène, un radical hydroxyle, un groupement acétoxy $CH_3COO$-, un groupement alkoxy inférieur en $C_1$-$C_5$ ou une chaine alkylènedioxy $-O-(CH_2)_n-O-$ dans laquelle n peut prendre les valeurs 1 ou 2, et $R_4$ peut représenter un atome d'hydrogène, un radical alkyle inférieur en $C_1$-$C_5$, ou un noyau phényle éventuellement substitué par des radicaux ou groupements $R_1$, $R_2$, $R_3$, de même nature que ceux mentionnés ci-dessus, et R′ représente un atome d'hydrogène ou un radical méthyle, et ses dérivés physiologiquement acceptables pour la fabrication d'un médicamant destiné au traitement des vessies hyperactives ou instables.

EP 0 509 913 A1

La présente invention concerne l'utilisation de composés dont d'autres applications sont connues pour la fabrication d'un médicament destiné au traitement de patients présentant des vessies instables ou hyperactives ("hyper reflexic bladder").

Les vessies instables ou hyperactives sont des vessies qui pour des raisons diverses conduisent à des mictions fréquentes et urgentes qui peuvent aller jusqu'à l'incontinence urinaire, qui sur le plan psychologique et social constitue un grave handicap.

Les causes d'une telle instabilité du détrusor peuvent être d'origines multiples, particulièrement dues aux effets secondaires de maladies telle que les tumeurs cérébrales ou de la moëlle épinière, les atteintes traumatiques du système nerveux, les accidents cérébro-vasculaires, les maladies démyélinisantes ou dégénératives comme la sclérose en plaque, la maladie de Parkinson et les démences.

Les traitements connus de ces vessies hyperactives consistent, soit en un traitement par des compositions présentant des effets antispasmodiques ou anticholinergiques, tels le chlorure d'oxybutynine, l'imipramine ou le bromure de propanthéline, soit en une intervention chirurgicale pour dénerver le détrusor.

L'utilisation de composés anticholinergiques, bien qu'efficace, s'accompagne souvent de nombreux effets secondaires dont une sécheresse de la bouche très importante qui les rendent souvent difficilement acceptables pour les patients. Il existe certains cas où aucun traitement réellement acceptable de ce type de pathologie n'est possible.

C'est pourquoi il est particulièrement intéressant d'avoir mis en évidence les activités surprenantes de composés connus par ailleurs pour le traitement de pathologies différentes.

Ainsi, la présente invention concerne l'utilisation d'un composé de formule générale I :

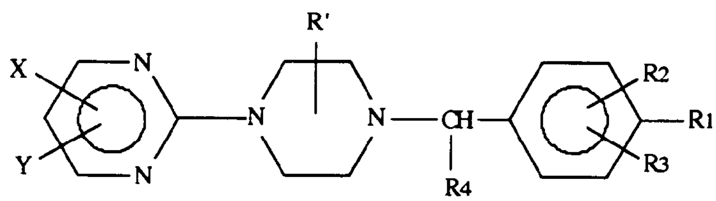

(I)

dans laquelle $R_1$, $R_2$, $R_3$, pris séparément ou en combinaison peuvent représenter un atome d'hydrogène, un radical hydroxyle, un groupement acétoxy $CH_3COO$-, un groupement alkoxy inférieur en $C_1$-$C_5$ ou une chaine alkylènedioxy $-O-(CH_2)_n-O-$ dans laquelle n peut prendre les valeurs 1 ou 2, et $R_4$ peut représenter un atome d'hydrogène, un radical alkyle inférieur en $C_1$-$C_5$, ou un noyau phényle éventuellement substitué par des radicaux ou groupements $R_1$, $R_2$, $R_3$, de même nature que ceux mentionnés ci-dessus, et R' représente un atome d'hydrogène ou un radical méthyle, ses dérivés physiologiquement acceptables et ses métabolites actifs pour la fabrication d'un médicament destiné au traitement des vessies hyperactives.

Par métabolites actifs on entend les produits de dégradation du composé de formule I actif dans le traitement des vessies hyperactives.

D'une manière préférentielle, on emploiera le piribédil connu en particulier pour ses propriétés vasodilatatrices, anti-ischémiques et anti-parkinsoniennes.

Parmi les déviés utilisables de ces produits, il faut citer les sels, notamment le méthanesulfonate pour le piribédil.

Compte-tenu de la pathologie à traiter, les formes galéniques les plus appropriés sont les formes orales, notamment les comprimés, ainsi que les formes galéniques retard, notamment les formes transdermiques.

Les comprimés sont généralement dosés en composé de formule générale I entre 15 et 60 mg.

Les formes injectables sont également préférées pour une administration intraveineuse ou parentérale.

Les formes et les doses utilisables peuvent varier entre 1 à 15 mg/kg par jour de principe actif, ces doses pouvant être adaptées en fonction de l'état du patient et de l'importance du trouble, de même que de son état d'évolution.

Les exemples ci-après permettent de mettre en évidence d'autres caractéristiques et avantages de la présente invention.

**EXEMPLE 1 : protocole de réalisation du test au piribédil.**

Le piribédil se présente sous la forme d'une solution injectable de 0,003 g/ml de méthanesulfonate de piribédil. La veille du test, le patient arrête éventuellement la prise des drogues antiparkinsoniennes, et ingère trois fois 20 mg de dompéridone, prise de 20 mg répétée le lendemain matin 2 heures avant le test.

Une perfusion est alors posée et une cystomanométrie et un profil urétral de référence sont effectués.

Tout au long du test, une surveillance des constance artérielles est effectuée. La solution de piribédil est injectée très lentement par voie intraveineuse.

Une cystomanométrie est effectuée toutes les 5 mn à 10 mn pendant 30 minutes. En particulier, une cystomanométrie doit être effectuée dès l'apparition de baillements et d'une somnolence.

Ce test a été effectué sur six patients présentant une vessie hyperactive liée soit à la maladie de Parkinson (I, II, III, et IV), soit à une sclérose en plaques (V et VI).

Les résultats des différents tests montrent en particulier pour le patient III en cystomanométrie une augmentation de capacité de la vessie de 180 à 260 $cm^3$ et pour le patient IV de 290 à 550 $cm^3$.

A la suite de ces tests, il a été décidé de traiter le patient VI par la prise de comprimés par voie orale contenant 0,05 g de piribédil par comprimé. Ce patient a été traité pendant 6 mois à la dose de 6 comprimés par jour. Les résultats surprenant montrent une nette amélioration clinique avec une diminution de l'incontinence urinaire et, après 6 mois, une vessie dont la capacité est stabilisée aux environs de 450 $cm^3$, alors qu'une mesure de référence avant le traitement avait montré une limite de 220 $cm^3$.

Au regard de ces résultats, de nouveaux tests ont été effectués sur trente cinq patients pour les tests intraveineux et sur soixante autres patients traités par du piribédil sous forme de comprimés.

**EXEMPLE II : Test au piribédil**

Le protocole suivi est identique à celui décrit dans l'exemple I.

La prise de dompéridone n'est pas indispensable à la réalisation du test et n'intervient que lorsque le patient présente des risques de vomissement.

Trente cinq patients porteurs d'une vessie hyperactive ont été explorés par tests intraveineux au piribédil, comportant une cystomanométrie avant injection suivie d'une série de cystomanométries après injection de 3 mg de piribédil en intra-veineuse lente.

Les pathologies de ces patients étaient : maladie de Parkinson, sclérose en plaques, affections neurovasculaires, atteinte du cône terminal et lésions traumatiques de la moelle épinière, neuropathie périphérique, traumatisés crâniens, instabilité vésicale idiopathique.

Dans 23 cas (66%) le test était positif : augmentation de la capacité vésicale de 30% ou plus par rapport à la capacité vésicale avant injection. Dans 7 de ces cas, l'augmentation de la capacité vésicale était supérieure à 50% (test très positif).

Dans 12 cas (33%) le test était négatif. Ces test négatifs étaient essentiellement représentés par les patients atteints d'une lésion médullaire traumatique (8 patients sur 12).

**EXEMPLE III : Patients ayant été traités par du piribédil per os sous forme de comprimés dosés à 20 mg ou 50 mg.**

Soixante patients porteurs d'une vessie hyperactive ont été suivis pendant 1 à 6 mois en étude ouverte, avec des doses quotidiennes de piribédil comprises entre 60 et 300 mg.

Les pathologies concernées étaient : maladie de Parkinson, sclérose en plaques, affections neuro-vasculaires, atteinte du cône terminal et lésions traumatiques de la moelle épinière, neuropathie périphérique, traumatisés crâniens, instabilité vésicale idiopathique, énurésie de l'enfant.

Le traitement a apporté un bénéfice dans 44 cas (73%) : disparition ou dimunition des fuite, meilleure possibilité de retenue, diminution de l'énurésie, diminution de la pollakiurie. Le traitement n'a pas eu d'effet dans 12 cas (20%).

Le traitement a du être interrompu dans 4 cas en raison d'effets secondaires intolérables (nausées, vomissements, vertiges). Sinon les effets secondaires ont été parfois gênants initialement dans 11 cas, sans entraîner l'interruption du traitement ou parfois simplement une réduction des doses. La prise de dompéridone a été nécessaire au long cours pour empêcher les vomissements chez quelques patients (7 cas).

## Revendications

1. Utilisation d'un composé de formule générale I

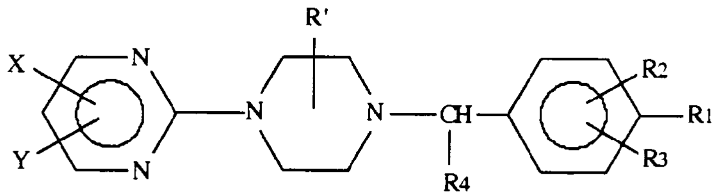

dans laquelle $R_1$, $R_2$, $R_3$, pris séparément ou en combinaison peuvent représenter un atome d'hydrogène, un radical hydroxyle, un groupement acétoxy $CH_3COO$-, un groupement alkoxy inférieur en $C_1$-$C_5$ ou une chaine alkylènedioxy -O-$(CH_2)_n$-O- dans laquelle n peut prendre les valeurs 1 ou 2, et $R_4$ peut représenter un atome d'hydrogène, un radical alkyle inférieur en $C_1$-$C_5$, ou un noyau phényle éventuellement substitué par des radicaux ou groupements $R_1$, $R_2$, $R_3$, de même nature que ceux mentionnés ci-dessus, et R' représente un atome d'hydrogène ou un radical méthyle, ses dérivés physiologiquement acceptables et ses métabolites actifs pour la fabrication d'un médicament destiné au traitement des vessies hyperactives ou instables.

2. Utilisation selon la revendication 1, caractérisée en ce que le composé de formule générale I est le piribédil ou ses sels physiologiquement acceptables.

3. Utilisation selon la revendication 2, caractérisée en ce que le composé de formule générale I est le méthanesulfonate de piribédil.

4. Utilisation selon l'une des revendications 1 à 3, caractérisée en ce que le médicament destiné au traitement des vessies hyperactives est un médicament sous une forme convenant pour l'administration par voie orale, intraveineuse ou parentérale.

5. Utilisation selon la revendication 4, caractérisée en ce que le médicament est sous la forme d'un comprimé.

6. Utilisation selon la revendication 5, caractérisée en ce que le comprimé est dosé en composé de formule générale I entre 15 et 60 mg.

7. Utilisation selon la revendication 4, caractérisée en ce que le médicament est sous une forme injectable.

Office européen des brevets

# RAPPORT PARTIEL DE RECHERCHE EUROPEENNE

qui selon la règle 45 de la Convention sur le brevet européen est consideré, aux fins de la procédure ultérieure comme le rapport de la recherche européenne

Numero de la demande

EP 92 40 1052

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | PATENT ABSTRACTS OF JAPAN, vol. 13, no. 208 (C-596), 16 mai 1989; & JP-A-1 026 517 (SANKYO CO., LTD) 27-01-1989<br>* Résumé en entier *<br>--- | 1-7 | A 61 K 31/505 |
| Y | LA REVUE DU PRATICIEN, vol. 36, no. 5, 21 janvier 1986, pages 237-241; D. LAPLANE: "Pour la pratique... on retiendra..."<br>* Page 240 *<br>--- | 1-7 | |
| Y | EUR. UROL., vol. 3, no. 1, 1977, pages 1-6; M. CAINE: "The importance of adrenergic receptors in disorders of micturition"<br>* Le document en entier *<br>--- -/- | 1-7 | |

DOMAINES TECHNIQUES RECHERCHES (Int. Cl.5)

A 61 K

## RECHERCHE INCOMPLETE

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.

Revendications ayant fait l'objet de recherches complètes: 2,3

Revendications ayant fait l'objet de recherches incomplètes: 1,4-7

Revendications n'ayant pas fait l'objet de recherches:

Raison pour la limitation de la recherche:

Aucune définition des valeurs prises par les variables X et Y (revendication 1) n'a été donnée, ni dans les revendications ni dans la description ou les exemples. Par conséquent la recherche ne les a pas inclues (X=Y=H) mais l'interprétation de la valeur des documents retrouvés a été large.

| Lieu de la recherche | Date d'achèvement de la recherche | Examinateur |
|---|---|---|
| LA HAYE | 07-07-1992 | GAC G. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P0408)

Office européen des brevets

# RAPPORT PARTIEL DE RECHERCHE EUROPEENNE

Numero de la demande

EP 92 40 1052

| DOCUMENTS CONSIDERES COMME PERTINENTS | | | CLASSEMENT DE LA DEMANDE (Int. Cl.5) |
|---|---|---|---|
| Catégorie | Citation du document avec indication, en cas de besoin, des parties pertinentes | Revendication concernée | |
| Y | EUROPEAN JOURNAL OF PHARMACOLOGY, vol. 52, 1978, pages 99-107, Elsevier/North-Holland Biomedical Press; M. LAUBIE et al.: "Inhibitory effects of piribedil on adrenergic neurotransmission"<br>* Le document en entier *<br>--- | 1-7 | |
| A | ANNALS OF CLINICAL RESEARCH, vol. 12, no. 1, février 1980, pages 1-4; S. VAIDYANATHAN et al.: "Role of dopamine receptors in vesicourethral function. A urodynamic study with dopamine receptor antagonist metoclopramide"<br>* Le document en entier *<br>--- | 1-7 | DOMAINES TECHNIQUES RECHERCHES (Int. Cl.5) |
| A | UROLOGY, vol. 9, no. 2, février 1977, pages 188-190; S. RAZ et al.: "Methyldopa in treatment of neorogenic bladder disorders"<br>* Le document en entier *<br>--- | 1-7 | |
| A | INVESTIGATIVE UROLOGY, vol. 15, no. 4, janvier 1978, pages 267-269, The Williams & Wilkins Co.; T. KOYANAGI et al.: "Reappraisal of the sympathetic role in the sphincteric urethra. Denervation supersensitivity of the urethra of the chronic neurogenic bladder to alpha-adrenergic drugs"<br>* Le document en entier *<br>--- | 1-7 | |
| A | BRITISH JOURNAL OF PHARMACOLOGY, vol. 71, no. 2, 1980, pages 513-518; C. CHEVILLARD et al.: "Prejunctional actions of piribedil on the isolated kidney of the rabbit: comparison with apomorphine"<br>* Le document en entier *<br>--- | 1-7 | |
| Y | POLISH JOURNAL OF PHARMACOLOGY AND PHARMACY, vol. 37, no. 3, 1985, pages 397-404; M. DODA et al.: "Dopaminergic inhibition of sympathetic activity in the cat"<br>* Le document en entier *<br>----- | 1-7 | |

EPO FORM 1503 03.82 (P0411)